# EUROPEAN PATENT SPECIFICATION

(11) **EP 3 463 232 B1**
(45) Date of publication and mention of the grant of the patent: **29.04.2020**
(21) Application number: 17726671.5
(22) Date of filing: 19.05.2017
(51) Int. Cl.: A61F 11/08, H04R 1/10

(54) **EAR PROTECTION DEVICE**
OHRENSCHUTZVORRICHTUNG
DISPOSITIF DE PROTECTION D'OREILLE

(30) Priority: 23.05.2016 GB 201609033
(43) Date of publication of application: 10.04.2019
(73) Proprietor: Minuendo AS, 0153 Oslo (NO)
(72) Inventor: PETTERSEN, Odd Kristen Østern, 7034 Trondheim (NO); KVALØY, Olav, 7034 Trondheim (NO)
(74) Representative: Dehns
(86) International application number: PCT/GB2017/051410
(87) International publication number: WO 2017/203218

(56) References cited:
- EP-A1- 1 313 417
- EP-A1- 1 629 808
- US-A1- 2011 103 605
- US-A1- 2012 082 335

## Description

This invention relates to ear protection used for reducing the intensity of sounds experienced by a user.

Exposure to high intensity noises can cause damage to a person's hearing. The damaging effects are increased when a person is frequently exposed to loud noises. In extreme cases, frequent exposure to loud noises can cause noise-induced hearing loss. Therefore, in order to protect hearing it is necessary to reduce the effects of continuous, intermittent and impact noises. As a result of the increasing awareness of the damaging effects of loud noises from industrial sources, there are various industry requirements for personnel to use ear protection. There are many situations in which personnel may be exposed to loud noises, for example when operating loud machinery. A common form of noise protection widely used are earplugs, these reduce the intensity of the sound entering a person's ears and thus reduce the damaging effects of high intensity noises.

There are two main types of earplugs that are commonly used: passive earplugs and active earplugs. Passive earplugs attenuate the intensity of all levels of sound equally, i.e. they provide a certain level of sound reduction, for example, a reduction of 20 dB. Passive earplugs come in various forms including: foam, silicon, flanged and custom moulded earplugs. Passive earplugs are typically inserted into a user's ear canal and, once in position, the material of the earplug acts to attenuate the sound which passes through it. As the incident sound passes through the earplug it is attenuated and subsequently passes out of the earplug into the user's ear canal where it is detected by the user.

Active earplugs comprise electronic components which enable the earplug to attenuate high intensity sounds. Active earplugs often comprise a passive earplug used in conjunction with a microphone on the external side of the earplug and a speaker on the internal side of the earplug. Active earplugs can employ control circuitry to apply different levels of attenuation at different times or different frequencies - known as 'adaptive attenuation'. Some systems contain circuitry that detects the incident sound and produces an out-of-phase signal that destructively interferes with the incident sound thus reducing the intensity of the incident sound as it propagates into the user's ear canal - this is often known as 'active noise cancelling' .

US 2012/082335 2012/00023302 discloses an earphone arrangement comprising a microphone and a sound transducer. A control circuit generates a valve control signal in response to the microphone signal to provide a variable attenuation.

When viewed from a first aspect the present invention provides a device for insertion into an ear canal of a mammalian subject and defining at least one sound path therethrough, as recited in claim 1.

Thus it will be seen by those skilled in the art that by providing at least two different levels of attenuation, which can be changed using the device itself, the device can be used in different situations which require different levels of attenuation without needing to physically remove the device. For example, if a user is operating heavy machinery the device may be operated in the second configuration in which the level of attenuation is higher, and then when a user stops using the heavy machinery, and the need for noise protection is reduced, the device may switch to the first configuration. This may, for example, allow the user to be able to hear quieter sounds such as conversations between other colleagues or other ambient noises.

Having to repeatedly remove and replace passive earplugs may cause users to avoid wearing them and thus potentially damage their hearing. The present invention may help to address this issue by removing the need to remove the device in order to switch between different levels of attenuation. As the device is able to switch between the two configurations upon receipt of a command signal, the device does not need to be physically removed as often and thus a user may therefore be more inclined to use it than risking their hearing.

It will also be understood that having two stable configurations of the mechanical attenuation components means that the device is effectively 'semi-active' in that the attenuating arrangement only uses electrical current to switch between the configurations but does not draw current during the rest of its operation. It will be appreciated that this is particularly advantageous as the device may only require electrical power for relatively short periods of time which may be relatively infrequent. The significant reduction in the amount of electrical power required to operate the device which this may provide could mean that such embodiments will have an improved battery life. This may mean that either a smaller battery can be used, potentially allowing the size of the device to be reduced, or alternatively it may simply extend the operational period of the device. Extending the battery life may be advantageous as it would reduce the requirement on a user to either recharge or replace the batteries in the device. Alternatively, providing a smaller battery may mean that the device can be lighter and more compact potentially meaning that it is more comfortable for the user. This is a significant improvement over active earplugs which constantly draw power in order operate their electronic control circuitry which monitors and adapts the earplug depending on the detected sound. Such active earplugs are well known to have a heavy power consumption and require frequent charging or battery replacement. It is also advantageous over arrangements which require power in order to maintain a particular level of attenuation e.g. a piezoelectric acoustic valve.

The device could, for example, operate in a default configuration which corresponds to the second configuration. After receiving the command signal to change from the second configuration to the first configuration, the device may be arranged to switch back automatically to the second configuration after a predetermined condition has been met, for example a period of time has elapsed. This may allow the user to be able to listen to quieter sounds such as a comment from a colleague for a short period of time before returning to the second configuration in which the level of attenuation is higher and thus providing more protection without the user having to remember to do so.

In a set of embodiments however said command signal causes the attenuating arrangement to change from said first configuration to said second configuration and receipt of a second command signal causes said attenuating arrangement to change from said second configuration to said first configuration. Such embodiments allow the device to be remotely controlled to switch between each configuration by a remote device. This may, for example, allow the user to have increased control over the operation of the device, allowing them to determine the configuration in which it is operating.

In a set of embodiments said attenuating arrangement has a third configuration having a third level of attenuation between the first and second levels. Such a set of embodiments is particularly advantageous as it may allow the device to be used in an increased number of situations. For example, such a set of embodiments would be highly useful in environments where there is intermediate-level noise, which is louder than an accepted base line noise but not as loud as significant impact noises. It is known that extended exposure to mid-level can cause hearing damage. Therefore, by having a third configuration, it means that the user may use the device to protect against the harmful mid-level noise. Increasing the number of situations in which the device can be used increases the chance of users making better use of the device and properly protecting their hearing. In another set of embodiments the attenuating arrangement may have any number of discrete levels of attenuation. Alternatively, in a set of embodiments the level of attenuation may be continuously variable. It will be appreciated by those skilled in the art that by providing a large number of attenuating arrangements or a continuously variable level of attenuation the device may be used in an increased number of situations/environments.

In a further set of embodiments where the attenuating arrangement has a third configuration the receipt of a third command signal causes said attenuating arrangement to change to said third configuration. This allows the device to be further controlled by an external device. In embodiments where the attenuating arrangement has a different number of levels of attenuation or a continuously variable level of attenuation a command signal may be provided from an external device to switch the attenuating arrangement into the configuration which corresponds to the level of attenuation.

It will be appreciated that the receiver within device may take various forms. However, in a set of embodiments said receiver is a radio-frequency (RF) receiver. A RF receiver is particularly advantageous as it does not require line of sight operation, and thus it may receive signals from a host of different devices, for example a user's mobile phone. In a further set of embodiments said receiver is Bluetooth™ compatible. This allows the device to receive commands from readily available electrical items, for example smart phones. In a further set of embodiments the receiver may comprise a Radio Frequency Identification Device (RFID) which receives power from an external device. Such a set of embodiments is further advantageous as the receiver is powered by the external device and thus does not draw power from a power supply within the device. This may help to increase the battery life of the device increasing its operational time.

In a set of embodiments the receiver operates in a sleep mode in which minimal power is drawn. This is particularly advantageous as the power consumption of the device is further reduced, thus extending the operational use of the battery.

The command signal is provided by a smart phone. It will be appreciated that using a smart phone is particularly advantageous as many people now own smart phones and thus it is likely that a user will have a smart phone that can be used with the device.

In a further set of embodiments the smart phone is provided with control software, for example in the form of a software application. This may allow the user to control the device easily. For example, it may allow the user to cause the device to switch between different configurations. The use of a smart phone, is also useful as they often comprise a microphone capable of detecting sounds. In a set of embodiments the smart phone comprises a microphone which is used as part of a sound level meter. It will be appreciated that this set of embodiments is particularly advantageous as the smart phone can be used to detect the intensity of incident sounds and may control the device accordingly. For example, if the smart phone detects a particularly loud sound it may send a command signal to the device in order to switch it into the second configuration. Conversely, if the smart phone detects low sound levels it may send a command signal to the device in order to switch it into the first configuration.

The attenuating arrangement may comprise any number of mechanical components which can affect the level of attenuation of the device. In a set of embodiments the attenuating arrangement comprises an electrical motor or actuator, for example a piezo-electric actuator or motor with a screw. In such a set of embodiments the electric motor or actuator may be employed to adjust the configuration of the mechanical arrangement between its stable states to provide the level of attenuation in such a way that the electric motor or actuator only need electrical current when moving between each configuration. In a set of embodiments the attenuating arrangement comprises a valve. It will be understood that the valve effectively controls the intensity of the sound which propagates through the device. The state of the valve therefore changes the level of attenuation. Therefore, adjusting the state of the valve effectively switches the device between different configurations.

In a set of embodiments the first level of attenuation is less than 25 dB, e.g.less than 10 dB, e.g. less than 5 dB. In embodiments where the first level of attenuation is less than 5 dB it is intended that whilst the device is in this configuration there is no deliberate attenuation of the incident sound (although a small degree of attenuation is inevitable).

The level of sound protection provided by the device is particularly important, particularly to meet prescribed health and safety requirements. The level of attenuation of the first configuration and the second configuration may be in a wide range of levels. However, in a set of embodiments said first and second attenuation levels differ by less than 25dB. This level of attenuation is considered to be an intermediate level of attenuation and as a result the device does not need to be inserted too far within the user's ear canal. As a result the device is suitable for situations which require mid-level attenuation. It is known that it is these specific mid-level noise environments where, due to the inconvenience of typical earplugs, a user may be more likely to take a risk and avoid wearing protection due to convenience. By providing a device that provides appropriate protection for these mid-level sound intensities, without the inconveniences of a typical earplug, a user may be more inclined to use the device and protect their hearing.

In a set of embodiments the device is in the form of an earplug adapted to conform tightly to the ear canal. It will be appreciated that an earplug which conforms tightly to the ear canal helps to prevent sound entering the ear canal without passing through the device, therefore helping to reduce the risk of high intensity sounds damaging the ear. This ultimately ensures that the device is able to control the sound level entering the ear canal. It is also important that the device conforms tightly to the ear canal to prevent the device from becoming loose or falling out. As the device may be used in environments where a user may be physically active the chances of the device becoming loose are increased, therefore this embodiment helps to reduce this risk of the device becoming loose and thus ensures contestant protection for the user.

Whilst the device may switch between configurations upon receipt of a command signal by the receiver it may also be possible to control the device directly from the device itself. In a set of embodiments the device comprises at least one button on the device and in operative connection with the attenuating arrangement. By touching the button a control signal may be sent to the attenuating arrangement. It will be appreciated that the provision of at least one button on the device may be advantageous as it may allow the user to easily switch between configurations without having to operate a further device. This may, for example, be useful as ear protection devices are often used in adverse conditions where controlling a further device may not be feasible.

Certain embodiments of the present invention will now be described, by way of example only, with reference to the accompanying drawings, in which:
Fig.1 shows a schematic view of a device in accordance with the present invention in a first configuration;
Fig. 2 shows a schematic view of the device of Fig. 1 in a second configuration; and
Fig. 3 shows a schematic of the control circuitry of the device of Figs 1 and 2.

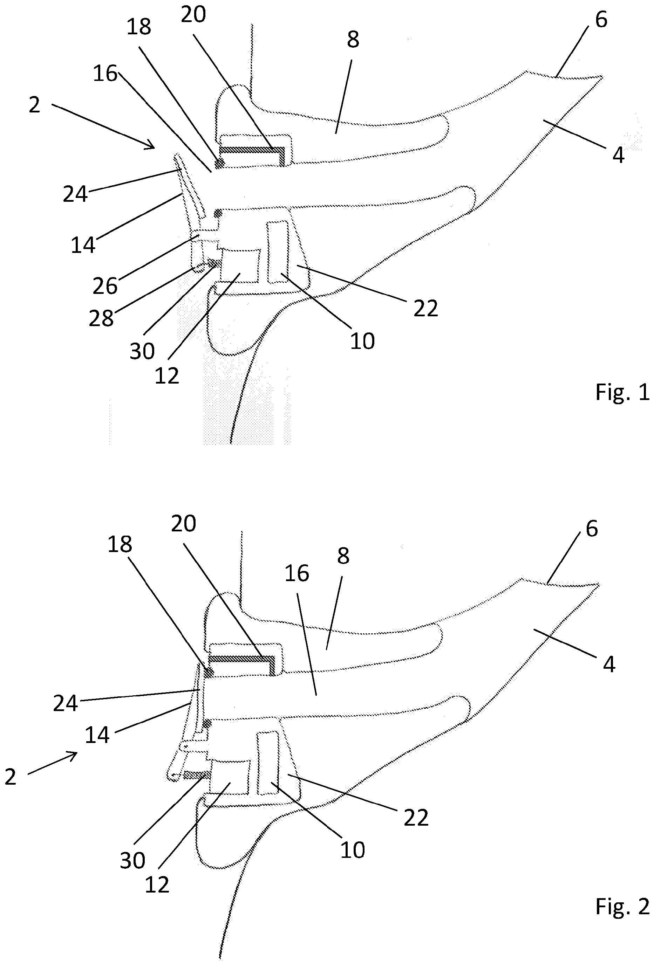

Figure 1 shows a device in accordance with the present invention in the form of an earplug 2. The earplug 2 is inserted into a user's ear canal 4. The user's ear canal 4 extends to the tympanic membrane 6. The tympanic membrane 6 vibrates in response to incident sound waves which travel along the ear canal 4.

The earplug 2 comprises a sealing plug 8, a control circuit 10, an electric actuator 12, a valve 14, and an internal channel 16 which runs through the sealing plug 8. A gasket 18 is provided at the opening of the internal channel 16 which is exposed to the external environment. The internal channel 16 and thus the opening may have any desired cross sectional shape. An acoustic leak channel 20 connects the outside environment to the internal channel 16. The acoustic leak channel 20 allows a certain amount of sound to pass into the channel 16 irrespective of the position of the valve 14 and provides pressure equalisation when the valve is closed. When the valve 14 is completely closed, the acoustic leak channel 20 controls the level of attenuation. The control circuit 10, electric motor 12 and acoustic leak channel 20 are provided in a segmented portion 22 of the sealing plug 8.

The valve 14 comprises a moveable member with a sealing face 24 for sealing against the gasket 18. The valve member 14 is pivotally mounted to a protruding member 26 which extends from the main body of the earplug 2. The distal end 28 of the valve 14 is acted on by an armature 30 which is driven by the actuator 12.

In order to protect their hearing, a user can insert the earplug 2 into their ear canal 4 to the point at which a good seal between the sealing plug 8 and ear canal 4 is achieved. The control circuit 10 then controls the level of attenuation of the earplug 2. In the embodiment seen in Figure 1 the earplug is in a first configuration which provides a first level of attenuation. The valve 14 is completely open and thus the earplug provides minimal attenuation of the sound which enters the user's ear canal 4. In order to achieve this configuration the control circuit 10 operates the motor 12 such that the actuator 12 pulls on distal end 28 of the valve 14 so that it pivots and moves the sealing face 24 away from the gasket 18. This opens up the channel 16 allowing sound to propagate along the channel 16 towards the tympanic membrane 6.

The level of attenuation provided by the earplug can be determined by comparing the sound pressure outside the ear canal 4 and the sound pressure inside the ear canal 4. This can be calculated by using the following formula: *Attenuation*[*dB*] = *Pₒᵤₜ*[*dB*] - *Pᵢₙ*[*dB*] where *Pᵢₙ*[*dB*] is the sound pressure inside the ear canal 4 measured in decibels and *Pₒᵤₜ*[*dB*] is the sound pressure outside the ear canal 4 also measuring in decibels. The relationship between the pressure in decibels and the pressure in Pascals is given by the following relationship: *P*[*dB*] = 20log(*p*/*p*₀), where *P*[*dB*] is the pressure in decibels, p is the pressure in Pascals, and *p*₀ is a reference pressure *p*₀ = 2 × 10⁻⁵ *Pascals.* If the pressures are measured in Pascals, the attenuation can be calculated using the following formula: *Attenuation* = *p*_{*out*/}*pᵢₙ*, where *pᵢₙ* is the pressure inside the ear canal measured in pascals, and *pₒᵤₜ* is the pressure outside the ear canal measured in pascals. This way of calculating attenuation gives a dimensionless number typically in the range from one to several thousand (if the attenuation is high).

Figure 2 shows the same earplug 2 in the second configuration which provides a second level of attenuation. In this configuration it will be appreciated that a significant portion of the sound which travels towards the user's ear will be attenuated. It is envisaged that the valve 14 is able to significantly reduce the sound passing through the valve 14 directly into the internal channel 16 and thus the user's ear canal 4. Therefore, in this configuration, sound which can enter the user's ear canal 4 is sound which has travelled via the acoustic leak channel 20 and the sound which has been attenuated by the valve 14. The acoustic leak channel 20 is designed such that it attenuates sound. As a result the sound intensity which can propagate through the acoustic leak channel 20 is significantly less than the sound which can propagate through the earplug 2 when the valve 14 is opened.

In order for the earplug 2 to operate in this second configuration, the actuator 12 drives the armature 30 to push on the distal end 28 of the valve 14 so that the end portion 24 is pivoted to seal against the gasket 18.

It will be appreciated that whilst Figures 1 and 2 only show two configurations in which two distinct levels of attenuation are achieved, it is envisaged that other levels of attenuation could be achieved. For example, the valve 14 may be positioned so as to be proximal to the gasket 18 but not forming a complete seal. This configuration may provide a third level of attenuation in between the levels achieved by the configurations seen in Figures 1 and 2. It will be appreciated that there is a continuous range of positions of the valve 14 which will be equivalent to different levels of attenuation and thus a range of different attenuation levels may be achieved using this example embodiment.

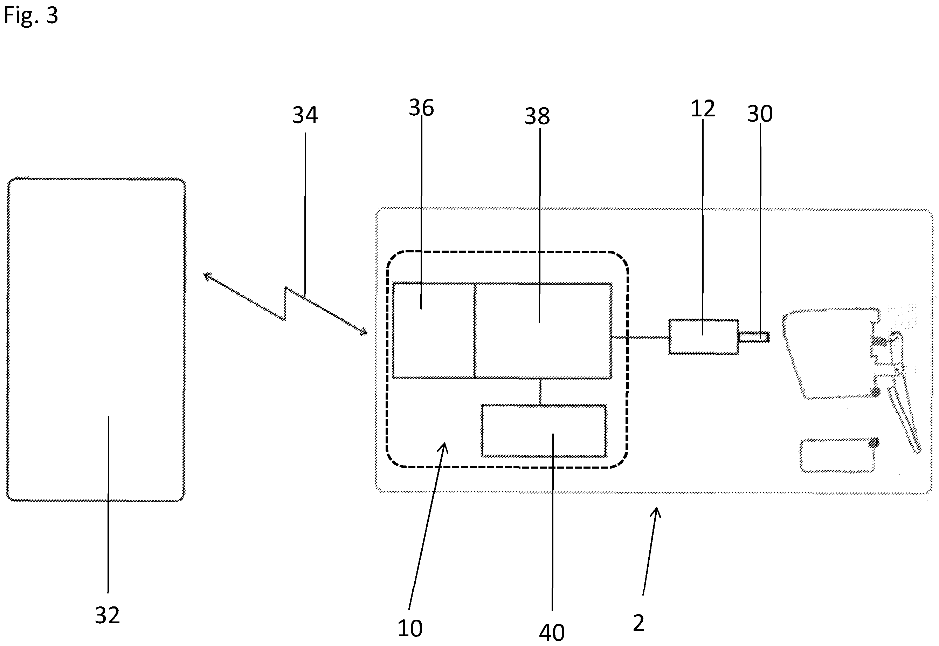

Figure 3 shows a schematic diagram of the earplug 2 interacting with an external device, for example a smart phone 32. The earplug 2 and smart phone 32 communicate via a radio link 34 which could, for example, be a Bluetooth™ connection. The control circuit 10, within the earplug 2, comprises a receiver unit 36, a control system 38 and a battery circuit 40. The battery circuit 40 powers the control system 38 and receiver unit 36. The control system 38 controls the output of the actuator 12 which moves the armature 30 which controls the position of the valve 14.

During use, the earplug 2 may receive a radio signal 34 from the smartphone 32 to instruct the earplug to operate in a second configuration equivalent to a second level of attenuation. In this case, the receiver 36 receives this radio signal which is subsequently interpreted by the control system 38. The actuator 12 is then driven so as to cause the valve 14 to close and thus the earplug 2 attenuates incoming sound at the second level of attenuation. In contrast, if a radio signal 34 is received from the smart phone 32 to instruct the earplug to operate in a first configuration in which the level of attenuation is lower, this can once again be received by receiver 36, interpreted by control circuit 38 and cause the motor 12 to be driven as to move the valve 14 into the open position allowing sound to freely travel into the user's ear canal 4.

A user may have the earplug 2 in their ear and have the earplug 2 in a first configuration in which the valve 2 is open (see Fig. 1). When a user enters a noisy environment, for example one in which power tools are being used, they may switch the earplug 2 to the second configuration in which the valve 2 is closed (see Fig. 2). When making this transition between different environments the user may, for example, press a button on the mobile device 32 in order to provide a command to switch between the two different configurations.

In other embodiments, the mobile device 32 may comprise a microphone and sound processing software. In such embodiments the microphone may detect the sound intensity in the environment in which it is in. As the mobile device 32 is likely to be carried by the user it will detect the sound intensity in the environment proximal to the user. The sound processing software can then measure the intensity of the sound and compare it to predetermined base levels. If the sound is determined to be above a predetermined base level the mobile device 32 may provide a command signal to the earplug 2 to switch it into the second configuration. The sound processing software may also monitor the detected sound levels and assess any frequent sounds which may be below the base level but may still cause damage to the user's hearing due to their repetitive nature. The mobile device 32 may then cause the earplug 2 to switch to the second configuration if such conditions are detected.

Once the earplug 2 is in a configuration appropriate for the detected sound levels, the mobile device 32 may continue to monitor the sound intensity in the environment in which the user is present. If/when the mobile device 32 detects that the sound conditions have changed, the mobile device 32 may provide a command signal to switch the earplug 2 into the first configuration. This ability to constantly monitor the sound conditions via the mobile device is advantageous as it does not require power consumption from the earplug 2 itself.

It will also be appreciated that there may be communication from the receiver 36 to the smartphone 32. For example the receiver may relay various pieces of operational state information to the smartphone, for example battery status information. This information may then be used by the smartphone and be used to indicate to the user when the batteries need replacing or recharging.

It will be appreciated that whilst in the example shown a motor 12 drives a valve 14 to open and close an internal channel 16, various other mechanisms could be employed.

## Claims

1. A device (2) for insertion into an ear canal (4) of a mammalian subject and defining at least one sound path therethrough, the sound path having an attenuating arrangement therein which has a first configuration having a first level of attenuation and a second configuration having a second level of attenuation, higher than the first level, the attenuating arrangement being such that upon application of an electrical control signal thereto it is caused to change between said first and second configurations; the attenuating arrangement being stable in both first and second configurations such that the attenuating arrangement only draws electrical current from a power source when changing between the first and second configurations; **characterised in that** the device further comprises a receiver (36) for receiving a command signal from a smartphone such that receipt of said command signal causes said electrical control signal to be applied to said attenuating arrangement.

2. The device (2) as claimed in claim 1 arranged such that said command signal causes the attenuating arrangement to change from said first configuration to said second configuration and receipt of a second command signal causes said attenuating arrangement to change from said second configuration to said first configuration.

3. The device (2) as claimed in claim 1 or 2 wherein said attenuating arrangement has a third configuration having a third level of attenuation between the first and second levels.

4. The device (2) as claimed in claim 2 wherein receipt of a third command signal causes said attenuating arrangement to change to said third configuration.

5. The device (2) as claimed in any preceding claim wherein said receiver (36) is a radio-frequency receiver.

6. The device (2) as claimed in any preceding claim wherein said receiver (36) is Bluetooth™ compatible.

7. The device (2) as claimed in any preceding claim wherein the receiver (36) comprises a Radio Frequency Identification Device which is configured to receive power from an external device.

8. The device (2) as claimed in any preceding claim wherein the receiver (36) is arranged to operate in a sleep mode in which minimal power is drawn.

9. The device (2) as claimed in any preceding claim wherein the smart phone (32) is provided with control software, for example in the form of a software application.

10. The device (2) as claimed in any preceding claim wherein the smart phone (32) comprises a microphone which is used as part of a sound level meter.

11. The device (2) as claimed in any preceding claim wherein the attenuating arrangement comprises an electrical motor or actuator (12).

12. The device (2) as claimed in claim 11 wherein the electric motor or actuator (12) is employed to adjust the mechanical arrangement between said first and second configurations in such a way that the electric motor or actuator only needs electrical current when moving between said configurations.

13. The device (2) as claimed in any preceding claim wherein the attenuating arrangement comprises a valve (14).

14. The device (2) as claimed in any preceding claim wherein the first level of attenuation is less than 25 dB.

15. The device (2) as claimed in any preceding claim wherein said first and second attenuation levels differ by less than 25dB.

16. The device (2) as claimed in any preceding claim comprising at least a portion thereof in a form of an earplug adapted to conform tightly to a user's ear canal (4).

17. The device (2) as claimed in any preceding claim comprising at least one button on the device and in operative connection with the attenuating arrangement.

## Patentansprüche

1. Vorrichtung (2) zum Einsetzen in einen Gehörgang (4) eines Säugetiersubjekts und zumindest einen Schallweg dort hindurch definierend, wobei der Schallweg eine Dämpfungsanordnung darin aufweist, die eine erste Konfiguration aufweist, die einen ersten Dämpfungspegel aufweist, und eine zweite Konfiguration, die einen zweiten Dämpfungspegel aufweist, der höher als der erste Pegel ist, wobei die Dämpfungsanordnung derart ist, dass bei einem Anlegen eines elektrischen Steuersignals daran bewirkt wird, dass sie zwischen der ersten und der zweiten Konfiguration wechselt; wobei die Dämpfungsanordnung in beiden, der ersten und der zweiten Konfiguration stabil ist, sodass die Dämpfungsanordnung nur dann elektrischen Strom von einer Stromquelle zieht, wenn sie zwischen der ersten und der zweiten Konfiguration wechselt; **dadurch gekennzeichnet, dass** die Vorrichtung weiter einen Empfänger (36) zum Empfangen eines Befehlssignals von einem Smartphone umfasst, sodass ein Empfang des Befehlssignals bewirkt, dass das elektrische Steuersignal an die Dämpfungsanordnung angelegt wird.

2. Vorrichtung (2) nach Anspruch 1, die angeordnet ist, sodass das Befehlssignal bewirkt, dass die Dämpfungsanordnung aus der ersten Konfiguration in die zweite Konfiguration wechselt, und ein Empfang eines zweiten Befehlssignals bewirkt, dass die Dämpfungsanordnung aus der zweiten Konfiguration in die erste Konfiguration wechselt.

3. Vorrichtung (2) nach Anspruch 1 oder 2, wobei die Dämpfungsanordnung eine dritte Konfiguration aufweist, die einen dritten Dämpfungspegel zwischen dem ersten und dem zweiten Pegel aufweist.

4. Vorrichtung (2) nach Anspruch 2, wobei ein Empfang eines dritten Befehlssignals bewirkt, dass die Dämpfungsanordnung in die dritte Konfiguration wechselt.

5. Vorrichtung (2) nach einem vorstehenden Anspruch, wobei der Empfänger (36) ein Hochfrequenz-Empfänger ist.

6. Vorrichtung (2) nach einem vorstehenden Anspruch, wobei der Empfänger (36) Bluetooth™-kompatibel ist.

7. Vorrichtung (2) nach einem vorstehenden Anspruch, wobei der Empfänger (36) eine RFID-Vorrichtung umfasst, die dazu konfiguriert ist, Strom von einer externen Vorrichtung zu erhalten.

8. Vorrichtung (2) nach einem vorstehenden Anspruch, wobei der Empfänger (36) angeordnet ist, in einem Schlafmodus zu arbeiten, in dem minimaler Strom gezogen wird.

9. Vorrichtung (2) nach einem vorstehenden Anspruch, wobei das Smartphone (32) mit einer Steuersoftware, beispielsweise in der Form einer Software-Anwendung, bereitgestellt ist.

10. Vorrichtung (2) nach einem vorstehenden Anspruch, wobei das Smartphone (32) ein Mikrofon umfasst, das als Teil eines Schallpegelmessers verwendet wird.

11. Vorrichtung (2) nach einem vorstehenden Anspruch, wobei die Dämpfungsanordnung einen elektrischen Motor oder Aktor (12) umfasst.

12. Vorrichtung (2) nach Anspruch 11, wobei der elektrische Motor oder Aktor (12) dazu eingesetzt wird, die mechanische Anordnung zwischen der ersten und der zweiten Konfiguration derart anzupassen, dass der elektrische Motor oder Aktor nur dann elektrischen Strom benötigt, wenn er zwischen den Konfigurationen verfährt.

13. Vorrichtung (2) nach einem vorstehenden Anspruch, wobei die Dämpfungsanordnung ein Ventil (14) umfasst.

14. Vorrichtung (2) nach einem vorstehenden Anspruch, wobei der erste Dämpfungspegel weniger als 25 dB beträgt.

15. Vorrichtung (2) nach einem vorstehenden Anspruch, wobei der erste und der zweite Dämpfungspegel um weniger als 25 dB voneinander abweichen.

16. Vorrichtung (2) nach einem vorstehenden Anspruch, die zumindest einen Abschnitt davon in einer Form eines Gehörschutzstöpsels umfasst, der dazu eingerichtet ist, sich eng an einen Gehörgang (4) eines Benutzers anzupassen.

17. Vorrichtung (2) nach einem vorstehenden Anspruch, die mindestens einen Knopf auf der Vorrichtung und in Wirkverbindung mit der Dämpfungsanordnung umfasst.

## Revendications

1. Dispositif (2) destiné à être introduit dans un canal auditif (4) d'un sujet mammifère et définissant au moins un trajet sonore le traversant, le trajet sonore ayant un agencement d'atténuation en son sein qui a une première configuration ayant un premier niveau d'atténuation et une deuxième configuration ayant un deuxième niveau d'atténuation, supérieur au premier niveau, l'agencement d'atténuation étant tel que lors de l'application d'un signal de commande électrique à ce dernier, il est amené à changer entre lesdites première et deuxième configurations ; l'agencement d'atténuation étant stable à la fois dans les première et deuxième configurations de telle sorte que l'agencement d'atténuation attire uniquement le courant électrique provenant d'une source d'alimentation lorsqu'il change entre les première et deuxième configurations ; **caractérisé en ce que** le dispositif comprend en outre un récepteur (36) destiné à recevoir un signal d'instruction provenant d'un téléphone intelligent tel qu'une réception dudit signal d'instruction entraîne une application du signal de commande électrique audit agencement d'atténuation.

2. Dispositif (2) selon la revendication 1, agencé de sorte que ledit signal d'instruction entraîne un changement de l'agencement d'atténuation de ladite première configuration à ladite deuxième configuration et une réception d'un second signal d'instruction entraîne un changement dudit agencement d'atténuation de ladite deuxième configuration à ladite première configuration.

3. Dispositif (2) selon la revendication 1 ou 2, dans lequel ledit agencement d'atténuation a une troisième configuration ayant un troisième niveau d'atténuation entre les premier et deuxième niveaux.

4. Dispositif (2) selon la revendication 2, dans lequel la réception d'un troisième signal d'instruction fait changer ledit agencement d'atténuation vers ladite troisième configuration.

5. Dispositif (2) selon l'une quelconque des revendications précédentes, dans lequel ledit récepteur (36) est un récepteur radiofréquence.

6. Dispositif (2) selon l'une quelconque des revendications précédentes, dans lequel ledit récepteur (36) est compatible Bluetooth™.

7. Dispositif (2) selon l'une quelconque des revendications précédentes, dans lequel le récepteur (36) comprend un dispositif d'identification radiofréquence qui est configuré pour recevoir de l'énergie d'un dispositif externe.

8. Dispositif (2) selon l'une quelconque des revendications précédentes, dans lequel le récepteur (36) est agencé pour fonctionner dans un mode de veille dans lequel une puissance minimale est attirée.

9. Dispositif (2) selon l'une quelconque des revendications précédentes, dans lequel le téléphone intelligent (32) est doté d'un logiciel de commande, par exemple sous la forme d'une application logicielle.

10. Dispositif (2) selon l'une quelconque des revendications précédentes, dans lequel le téléphone intelligent (32) comprend un microphone qui est utilisé comme partie d'un sonomètre.

11. Dispositif (2) selon l'une quelconque des revendications précédentes, dans lequel le dispositif d'atténuation comprend un moteur électrique ou un actionneur (12).

12. Dispositif (2) selon la revendication 11, dans lequel le moteur électrique ou l'actionneur (12) est employé pour ajuster l'agencement mécanique entre lesdites première et deuxième configurations de sorte que le moteur électrique ou l'actionneur n'a besoin que d'un courant électrique quand il passe entre lesdites configurations.

13. Dispositif (2) selon l'une quelconque des revendications précédentes, dans lequel le dispositif d'atténuation comprend une vanne (14).

14. Dispositif (2) selon l'une quelconque des revendications précédentes, dans lequel le premier niveau d'atténuation est inférieur à 25 dB.

15. Dispositif (2) selon l'une quelconque des revendications précédentes, dans lequel lesdits premier et deuxième niveaux d'atténuation diffèrent de moins de 25 dB.

16. Dispositif (2) selon l'une quelconque des revendications précédentes, comprenant au moins une partie de celui-ci sous la forme d'une oreillette adaptée pour se conformer étroitement au canal auditif (4) d'un utilisateur.

17. Dispositif (2) selon l'une quelconque des revendications précédentes, comprenant au moins un bouton sur le dispositif et en connexion fonctionnelle avec l'agencement d'atténuation.
